(19) Europäisches Patentamt
European Patent Office
Office européen des brevets

(11) **EP 4 600 639 A1**

(12) **EUROPEAN PATENT APPLICATION**

(43) Date of publication:
**13.08.2025 Bulletin 2025/33**

(51) International Patent Classification (IPC):
***G01N 27/30*** *(2006.01)*     ***G01N 33/543*** *(2006.01)*
***G01N 33/487*** *(2006.01)*     ***A61B 5/00*** *(2006.01)*

(21) Application number: **24156341.0**

(22) Date of filing: **07.02.2024**

(52) Cooperative Patent Classification (CPC):
**G01N 27/3275; A61B 5/14; G01N 33/48707;
G01N 33/5438**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Xsensio SA**
**1015 Lausanne (CH)**

(72) Inventors:
• **Grammoustianou, Aristea**
**1015 Lausanne (CH)**
• **Saeidi, Ali**
**1015 Lausanne (CH)**

(74) Representative: **Vesterinen, Jussi Tapio**
**LUMI IP GmbH**
**Rodtmattstrasse 45**
**3014 Bern (CH)**

(54) **ELECTROCHEMICAL IMPEDANCE SYSTEM AND METHOD FOR REAL-TIME MONITORING OF BIOCHEMICAL INTERACTIONS AND/OR SENSING BIOMARKERS**

(57) An electrochemical impedance system (1) and method are provided for monitoring biochemical interactions and/or sensing biomarkers in a biofluid. The system (1) comprises at least one electrochemical cell (2) with electrodes configured to come in contact with the biofluid, and a potentiostat (3) for controlling operational conditions of the electrochemical cell (2) and for measuring reactions in the electrochemical cell (2). The potentiostat (3) applies an alternating voltage or current input with a constant frequency during one experiment to the electrochemical cell (2), and measures an output signal of the electrochemical cell (2) to thereby measure at least an initial impedance value of the electrochemical cell (2) at the beginning of a reaction taking place in the electrochemical cell (2), and an end impedance value of the electrochemical cell (2) at the end of the reaction to measure an impedance change of the electrochemical cell (2) attributable to the reaction.

Fig. 1

**Description**

TECHNICAL FIELD

The present invention relates to a method for real-time monitoring of biochemical interactions and/or sensing biomarkers by using an electrochemical impedance system, where a constant-frequency alternating voltage or current is applied to one or more of the working electrodes. The present invention also relates to an electrochemical impedance system configured to carry out real-time monitoring of biochemical interactions and/or to sensing biomarkers in real-time.

BACKGROUND OF THE INVENTION

Biosensors are analytical devices that convert biological interactions into digital signals. In recent years, they have proved their potential to find applications in the fields of medical diagnostics, food quality and safety, environmental monitoring and biodefense. According to the International Union of Pure and Applied Chemistry (IUPAC) a biosensor is an analytical device which provides quantitative or semi-quantitative information using a biological or biologically derived biorecognition element that targets a specific bio-analyte and that is in contact with a physicochemical transduction element. The biorecognition element can be made of a biological material (e.g. enzymes, antibodies, nucleic acids or cell receptors), a biologically derived material, such as an aptamer or a recombinant antibody, or even a bio-mimic material (molecularly imprinted polymers and synthetic catalysts). The biorecognition interaction between the biorecognition element and the analyte is converted by the transducer into a digital signal, which, in turn, is interpreted by a computer.

Over the past years, efforts have been focused on the development of biosensors that will be able to detect disease biomarkers and will be applied in health monitoring, disease diagnosis, and disease treatment, at the point-of-care. These biosensors emerge as alternatives to conventional, laboratory-based diagnostic techniques: chromatographic, spectroscopic, immunological-based (such as enzyme-linked immunosorbent assay (ELISA) and lateral flow immunoassays), and nucleic acid-based techniques (polymerase chain reaction (PCR), real-time PCR), etc. Electrochemical biosensors, in particular, are being constantly studied and their reported applications cover a wide range in the field of healthcare, including the detection of foodborne pathogens, bacterial infections, respiratory diseases, cancer, clinical diagnosis, etc. Electrochemical biosensors are classified into potentiometric, amperometric, and impedimetric sensors. Impedimetric sensors, in particular, can detect a biorecognition event occurring at the sensing surface, through the change of the interfacial properties.

Few publications have reported the application of impedimetric biosensors for the analysis of clinically relevant samples. Most of the electrochemical impedance spectroscopy (EIS)-based sensors rely on Faradaic impedance, and require the presence of a redox probe, hence, they are automatically classified as labelled sensors. Lasserre et al. in a publication "SARS-CoV-2 aptasensors based on electrochemical impedance spectroscopy and low-cost gold electrode substrates", Anal Chem. 94, 2126-2133 (2022), for example, employed an impedimetric biosensor for the detection of the SARS-CoV-2 spike protein, using a SARS-CoV-2 truncated aptamer. They used ferricyanide as the redox couple and the EIS measurement was taken after 15 minutes of incubation of the SARS-CoV-2 protein with the aptamer. The ferricyanide redox couple was also used by Vasantham et al. in a publication "Paper based point of care immunosensor for the impedimetric detection of cardiac troponin I biomarker", Biomed Microdevices 22, 6 (2019). They reported the detection of cardiac troponin I (cTnI) with an EIS biosensor that achieved a limit of detection (LOD) of 0.05 ng/mL. Despite the low LOD reported, the fact that these biosensors require a redox probe complicates their application in real-life, point-of-care settings, outside a traditional laboratory.

Non-Faradaic EIS sensors on the other hand, do not need a redox probe and, hence, they are considered to be label-free. The vast majority of publications employing the non-Faradaic type of EIS sensors use Nyquist plots which are extracted by measuring the impedance in a wide range of frequencies to describe the change of impedance occurring when a biorecognition interaction takes place on the sensing surface. Although this type of measurement is viewed as a label-free measurement, measurements according to a significant number of publications use signal amplification to increase detection sensitivity. Signal amplification can take the form of nanotechnology-based and biotechnology-based strategies, such as nanotags, nanocatalysis, nanocarriers, assembly-based, and polymerase-based DNA amplification strategies.

Existing multi-step EIS-based biosensors, which are often highly complex, present limitations in terms of their integration and miniaturisation. Research efforts have been made to simplify the non-Faradaic EIS biosensors. Aydin et al. in a publication "An impedimetric immunosensor for highly sensitive detection of IL-8 in human serum and saliva samples: a new surface modification method by 6-phosphonohexanoic acid for biosensing applications", Anal Biochem. 554, 44-52 (2018), employed an EIS sensor and swept frequencies from 50000 Hz to 0.05 Hz during measurements in order to detect interleukin-8 (IL-8) in human serum and saliva. No signal amplification or sample preconcentration was employed, and the LOD reported was 6 fg/mL. Nevertheless, the incubation time of IL-8 with the sensor was 45 minutes, which constrains its implementation in a point-of-care diagnostic device. The same group in a publication "Selective and ultrasensitive electrochemical immunosensing of NSE cancer biomarker in human serum using epoxy-substituted poly(pyrrole) polymer modified disposable ITO electrode", Sens Actuators B Chem., 306, 127613 (2020), presented the detection of a standard biomarker of lung cancer, the neuron specific enolase (NSE), using the same type of biosensor, and achieving the same LOD (6.1 fg/mL). This time, they reduced the sensor's incubation time to 30 minutes, which still is prohibiting for a point-of-care biosensor.

SUMMARY OF THE INVENTION

It is an object of the present invention to overcome at least some of the problems identified above related to sensing biomarkers and/or monitoring biochemical interactions in an electrochemical cell.

According to a first aspect of the invention, there is provided a method for real-time monitoring of biochemical interactions and/or sensing biomarkers in a biofluid by using an electrochemical impedance system as recited in claim 1.

The proposed method offers various advantages. Specifically, the ability to operate at a constant frequency facilitates real-time monitoring of biochemical interactions. The system sensitivity can be adjusted based on the operational frequency to align with the biochemical reaction. The shortened incubation time, a unique characteristic of this invention, sets it apart from conventional EIS-based methods that, unlike this approach, utilise Nyquist and/or Bode plots to illustrate changes in impedance. Consequently, the method is characterised by its speed (incubation time less than 5 minutes) and the production of impedance-versus-time plots, providing real-time impedance data. Moreover, the sensor utilised in this method can be either label-free or labelled.

According to a second aspect of the invention, there is provided an electrochemical impedance system for monitoring biochemical interactions and/or sensing biomarkers in a biofluid as recited in claim 9.

Other aspects of the invention are recited in the dependent claims attached hereto.

BRIEF DESCRIPTION OF THE DRAWINGS

Other features and advantages of the invention will become apparent from the following description of non-limiting example embodiments, with reference to the appended drawings, in which:

• Figure 1 schematically illustrates a first example electrochemical impedance system forming a single sensor, two-electrode system, where the teachings of the present invention can be implemented;

• Figure 2 schematically illustrates part of a second example electrochemical impedance system forming a single sensor, three-electrode system where the teachings of the present invention can be implemented;

• Figure 3 schematically illustrates part of a third example electrochemical impedance system forming a double sensor system with two electrodes per sensor, where the sensors form separate electrochemical cells, where the teachings of the present invention can be implemented;

• Figure 4 schematically illustrates part of a fourth example electrochemical impedance system forming a double sensor system with three electrodes per sensor, where the sensors form separate electrochemical cells, where the teachings of the present invention can be implemented;

• Figure 5 schematically illustrates part of a fifth example electrochemical impedance system forming a double sensor system with two electrodes per sensor, where the sensors form a single electrochemical cell, where the teachings of the present invention can be implemented;

• Figure 6 schematically illustrates part of a sixth example electrochemical impedance system forming a double sensor system with three electrodes per sensor, where the sensors form a single electrochemical cell, where the teachings of the present invention can be implemented;

• Figure 7 shows an example Bode plot for the electrochemical cell shown in Figure 1, as well as an electrical circuit model of the electrochemical cell; and

• Figures 8a and 8b show a flow chart illustrating an example method for monitoring biochemical interactions and/or sensing biomarkers in a biofluid by using an electrochemical impedance system.

DETAILED DESCRIPTION OF EMBODIMENTS OF THE INVENTION

Some embodiments of the present invention will now be described in detail with reference to the attached figures. As utilised herein, "and/or" means any one or more of the items in the list joined by "and/or". As an example, "x and/or y" means any element of the three-element set {(x), (y), (x, y)}. In other words, "x and/or y" means "one or both of x and y." As another example, "x, y, and/or z" means any element of the seven-element set {(x), (y), (z), (x, y), (x, z), (y, z), (x, y, z)}. In other words, "x, y and/or z" means "one or more of x, y, and z." Furthermore, the term "comprise" is used herein as an open-ended term. This means that the object encompasses all the elements listed, but may also include additional, unnamed elements. Thus, the word "comprise" is interpreted by the broader meaning "include", "contain" or "comprehend". Identical or corresponding functional and structural elements which appear in the different drawings are assigned the same reference numerals. It is to be noted that the use of words "first", "second" and "third", etc. may not imply any kind of particular order or hierarchy unless this is explicitly or implicitly made clear in the context. Moreover, the word "signal" may be interpreted in the present description so refer to a function that conveys information about a phenomenon, but it does not necessarily include any encoded information unless this is explicitly or implicitly made clear in the context.

The electrochemical impedance system as shown in the figures is a single-cell or dual-cell electrochemical impedance system developed for the real-time or substantially real-time monitoring and detection of biochemical interactions, employing electrodes as transducers. Depending on specific requirements, the system according to the examples below includes between 2 to 6 electrodes, which may be made of various materials, such as metals, oxides or two-dimensional (2D) materials, i.e. single-layer materials, such a graphene. At least some of these electrodes are designed to undergo functionalisation to enhance their capability for detecting specific target biomarkers or one or more analytes of interest. The analyte of interest may include molecular biomarkers, such as proteins, hormones, metabolites, nucleic acids, and/or whole cells. Measurements will be conducted using a potentiostat, also known as a galvanostat.

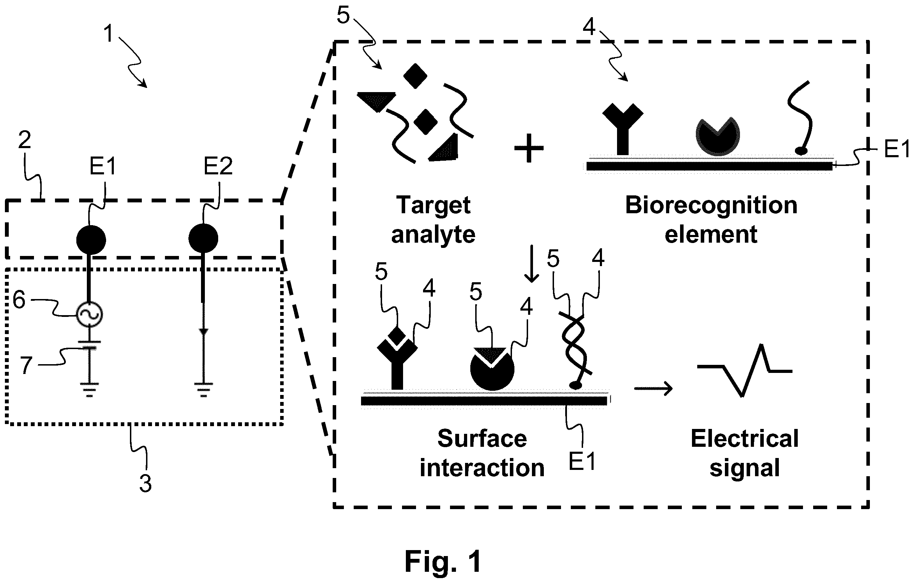

Figure 1 schematically shows a first example electrochemical impedance system 1 where the teachings of the present invention can be implemented. The system in this first configuration comprises an electrochemical cell 2, which is coupled to a control and measurement arrangement 3, which in this case is a potentiostat. However, any device or system that can provide and AC input signal and measures an AC signal precisely can be used as the control and measurement arrangement. The potentiostat 3 is operatively coupled to the electrochemical cell 2 to control operational conditions of the electrochemical cell and to measure reactions in the electrochemical cell. The electrochemical cell in this example comprises a first electrode E1, which in this example is a working electrode (WE), and a second electrode, which in this example is a counter electrode (CE), which may optionally operate as a combined counter electrode and a reference electrode. Electrodes within an electrochemical cell function as conductive interfaces linking an electronic circuit and an electrolyte. This arrangement allows for the monitoring of electrochemical reactions occurring on the electrode surface by assessing electrical and/or electrochemical properties of the electrode. In the present example, the working electrode E1 is functionalised with a biorecognition element or layer 4 to capture analytes 5 of interest from the electrolyte, also referred to as a biofluid or solution, which in this case is a liquid. At least in operation, the electrochemical impedance system thus comprises the biofluid. The biorecognition element 4 comprises, or is composed of biological material, or the biorecognition element comprises, or is composed of biologically derived material, or the biorecognition element comprises, or is composed of bio-mimic material, or the biorecognition element is composed of any combination of biological material, biologically derived material, and/or bio-mimic material. The biological material comprises one or more antibodies, enzymes, nucleic acids, and/or cell receptors, the biologically derived material comprises one or more aptamers, and/or recombinant antibodies, or the bio-mimic material comprises one or more molecularly imprinted polymers and/or synthetic catalysts.

The working electrode E1 is the electrode where in the present example the electrochemical reaction of interest takes place. It is the electrode at which the species being studied are either bonded, reacted, oxidized or reduced. The working electrode is typically the electrode under investigation, where we want to observe or control an electrochemical process. The reference electrode is used to control the potential of the electrochemical cell. It provides a stable and known electrochemical potential against which the potential of the working electrode can be compared. The counter electrode E2, also known as the auxiliary electrode, provides a pathway for the flow of current to or from the working electrode E1. It completes the electrical circuit in the electrochemical cell 2.

As shown in Figure 1, the potentiostat comprises a voltage source 6, which is an alternating voltage source $V_{AC}$, to provide an input voltage to the electrochemical cell, although an alternating current source $I_{AC}$ could be used instead to provide an input current to the electrochemical cell. In this example, the potentiostat includes a direct current (DC) source voltage 7, utilised to establish the DC bias of the working electrode. The primary function of this DC bias source is to uphold a predetermined potential at the working electrode.

The right-hand portion of Figure 1 illustrates the operation of the electrochemical cell 2. As shown, the target analytes 5 are captured by the biorecognition element 4. The biochemical interaction on the surface of the electrode changes the capacitive and/or resistive impedance of the electrochemical cell under test, which is measured by the potentiostat.

In operation, a continuous AC signal, with a frequency fixed during at least one experiment, is applied to the working electrode E1 or alternatively to the counter electrode E2, and the resulting signal, whether it is current or voltage, relative to the input signal, reflects the impedance of the electrochemical cell being tested. This methodology is employed for monitoring in real-time biochemical interactions occurring on the surface of the electrode which is functionalised. The selection of the fixed-frequency AC signal is made in accordance with the sensor's design. This specific frequency is chosen to optimise the capacitive and/or resistive response of the cell according to the target analyte and/or the type of the biochemical reaction thereby maximising system sensitivity. The process for optimising and selecting the operating frequency is later elaborated. Any interaction/reaction occurring on the electrode's surface that results in either the mass adsorption or generation of charges on the electrode will lead to a change in the capacitive and/or resistive response of the cell. These changes can be harnessed for the purpose of monitoring and detecting the target biomolecule and/or biochemical interactions.

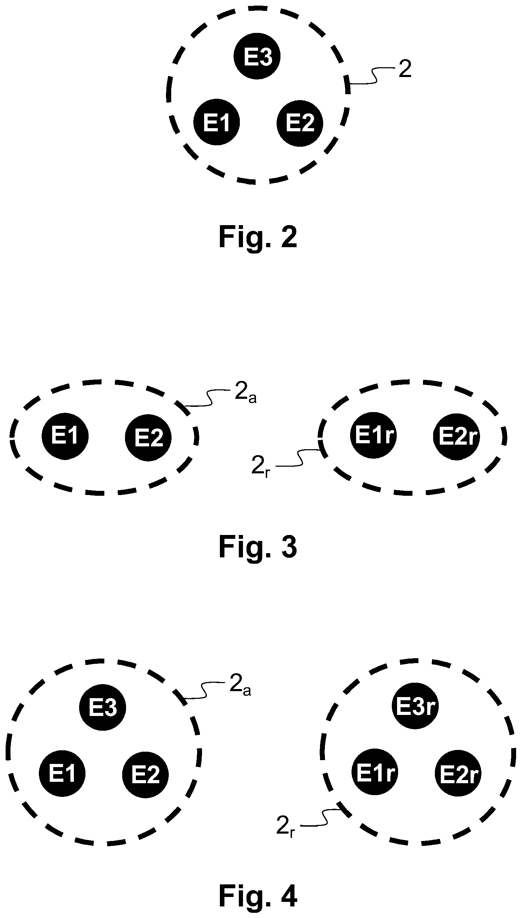

Figure 2 schematically shows a second example of electrochemical cell configuration where the teachings of the present invention can be implemented. The system according to this second configuration as shown in Figure 2 is a three-electrode system comprising a first electrode E1, which in this case is a working electrode, a second electrode E2, which in this case is a counter electrode, and a third electrode E3, which in this case is a reference electrode.

In the single-sensor set-up as shown in Figures 1 and 2, the biorecognition element 4 is applied to the working electrode E1 and/or to the counter electrode E2. In this electrochemical cell setup, a DC signal serves to establish the reference potential. An AC signal, which is either voltage or current, is applied to the working electrode E1 or counter electrode E2 at a constant frequency, and subsequently, the resulting signal, be it current or voltage, is recorded at the counter electrode E2 or working electrode E1, respectively. The distinction in phase and amplitude between the input and measured signals is employed to characterise or measure the impedance of the electrochemical cell 2. Depending on the specific analyte of interest, either the real or imaginary part of the impedance or both can be considered as the sensing signal. The roles of the working electrode E1 and counter electrode E2 are interchangeable in this context. While there are various methods for extracting the electrochemical cell's impedance, the results obtained through these methods are fundamentally similar. The electrochemical cell can use a common counter electrode and reference electrode as shown in Figure 1 or have a separate reference electrode as shown in Figure 2.

Figure 3 schematically shows a third example of electrochemical cell configuration where the teachings of the present invention can be implemented. In this third configuration the system forms a double sensor comprising a first electrochemical cell $2_a$, which in this case serves as an active or target sensor, and a second electrochemical cell $2_r$, which in this case serves as a reference sensor. The active sensor exhibits sensitivity to the target analyte, whereas the reference sensor remains insensitive to the target. The first electrochemical cell $2_a$ comprises a first working electrode E1 and a first counter electrode E2 operating in this case also as a first reference electrode, while the second electrochemical cell $2_r$ comprises a second working electrode E1r and a second counter electrode E2r operating in this case also as a second reference electrode. The electrochemical cells according to this configuration are two-electrode cells and individually form two-electrode sensors.

Figure 4 schematically shows a fourth example of electrochemical cell configuration where the teachings of the present invention can be implemented. In this fourth configuration the system forms a double sensor comprising a first electrochemical cell $2_a$, which in this case serves as an active or target sensor, and a second electrochemical cell $2_r$, which in this case serves as a reference sensor. The first electrochemical cell $2_a$ comprises a first working electrode E1, a first counter electrode E2, and a first reference electrode E3, while the second electrochemical cell $2_r$ comprises a second working electrode E1r, a second counter electrode E2r, and a second reference electrode E3r. The electrochemical cells according to this configuration are three-electrode cells and individually form three-electrode sensors.

The third and fourth configurations, as well as the other electrochemical cell configurations presented in the present description, primarily serve the purpose of detecting target biomolecules, such as proteins and hormones. To mitigate parasitic (non-specific) signals, simplify data analysis, and minimise errors, a reference sensor, i.e., the second electrochemical cell, is introduced alongside the active sensor $2_a$. These setups involve two parallel electrochemical cells, both exposed to, or in contact with the same biofluid. In one cell, which in this case is the first electrochemical cell, the biofunctional element is applied to the first working electrode E1 and/or the first counter electrode E2, rendering it sensitive to the target biomolecule (i.e., the receptors of the biorecognition element are able to capture the analyte of interest). In the other cell, which in this case is the second electrochemical cell, a non-specific biofunctional element or layer is applied to the second working electrode E1r and/or the second counter electrode E2r, making it insensitive to the target (i.e., the receptors of the biorecognition element are unable to capture the analyte of interest). The sensing signal would be $\Delta Z_{real}$ and/or $\Delta Z_{imaginary}$:

•

$$\Delta Z_{imaginary} = Z_{imaginary\_active} - Z_{imaginary\_reference};$$

and/or

•

$$\Delta z_{real} = Z_{real\_active} - Z_{real\_reference},$$

where $Z_{imaginary\_active}$ denotes the imaginary impedance value of the active (first) electrochemical cell, $Z_{imaginary\_reference}$ denotes the imaginary impedance value of the reference (second) electrochemical cell, $Z_{real\_active}$ denotes the real impedance value of the active (first) electrochemical cell, and $Z_{real\_reference}$ denotes the real impedance value of the reference (second) electrochemical cell.

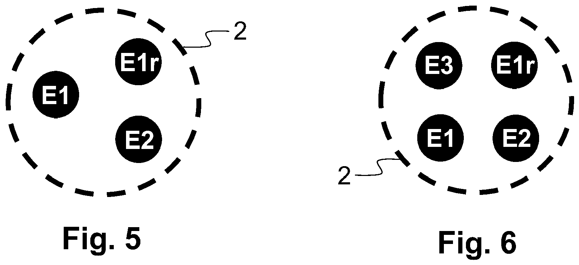

Figure 5 schematically shows a fifth example of electrochemical cell configuration where the teachings of the present invention can be implemented. In this fifth configuration the system forms a double sensor comprising only one electrochemical cell 2. The electrochemical cell 2 comprises a first working electrode E1, a second working electrode E1r, and a counter electrode E2 optionally operating also as a reference electrode. The electrochemical cell according to this configuration is a three-electrode cell. In this case, the active sensor comprises the first working electrode E1 and the counter electrode E2, while the reference sensor comprises the second working electrode E1r and the counter electrode E2.

Figure 6 schematically shows a sixth example of electrochemical cell configuration where the teachings of the present invention can be implemented. In this sixth configuration the system forms a double sensor comprising only one electrochemical cell 2. The electrochemical cell 2 comprises a first working electrode E1, a second working electrode E1r, a counter electrode E2, and a reference electrodeE3. The electrochemical cell according to this configuration is a four-electrode cell. In this case, the active sensor comprises the first working electrode E1, the counter electrode E2, and the reference electrode E3, while the reference sensor comprises the second working electrode E1r, the counter electrode E2, and the reference electrode E3.

The primary objective and underlying principle of the fifth and sixth configurations closely align with the third and fourth configurations, but the fifth and sixth configurations feature the following differences compared with the third and fourth configurations:

• Both the active and reference sensors are immersed in the same biofluid (single electrochemical cell).
• The counter electrode E2 and the reference electrode E3 are shared between both the active and reference sensors, leading to reduced complexity and a smaller surface area to manage.
• The first working electrode E1 is modified with the specific biofunctional element, while the second working electrode E2r is coated with the non-specific biofunctional element (although this setup is possible also in the third and fourth configurations but in these configurations the two sensors are in different electrochemical cells).
• Neither the counter electrode E2 nor the reference electrode E3 undergo any biofunctionalisation (although this holds true also for the electrodes of the reference sensor in the third and fourth configurations).

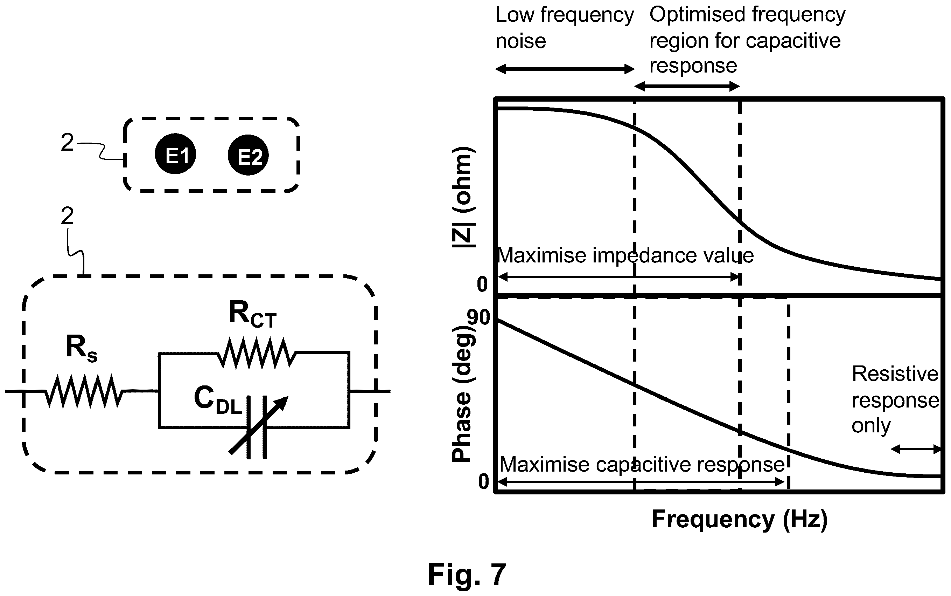

The working frequency of the real-time electrochemical impedance system 1 is optimised by measuring the bode plot as shown on the right-hand side of Figure 7, which is the frequency response, i.e., the changes in magnitude and phase as a function of frequency, of the electrochemical cell under test and considering the fact that: $Z_{imaginary}=|Z|\times sin(\varphi)$ and $Z_{real}=|Z|\times cos(\varphi)$, where $\varphi$ denotes the phase of the impedance. In order to enhance the sensitivity of the electrochemical impedance system, particularly with regard to the imaginary part of the signal, it is important to select a working frequency that maximises the capacitive response of the system. It is worth noting that in extremely low frequencies, low-frequency noise becomes dominant. Therefore, there exists a trade-off between maximising the capacitive signal and managing low-frequency noise to achieve an optimized signal-to-noise ratio (SNR), as visually illustrated in Figure 7.

In the electrical circuit model of the electrochemical cell 2 as shown on the left-hand side in Figure 7, Rs is the ohmic resistance of the solution, i.e., the biofluid, $R_{CT}$ is the charge transfer resistance of the respective electrode, and $C_{DL}$ is the capacitance of the electrochemical double layer, which is a structure that appears on the surface of an object when it is exposed to a fluid. Thus, in this case, an electrochemical double layer refers to the region of separation of charge that forms at the interface between an electrode and an electrolyte solution. This interface exists due to the difference in potential between the electrode and the electrolyte. Therefore, the total impedance of the electrode can be calculated as follows:

$$Z(\omega) = R_S + \frac{R_{CT}}{1+i\omega\tau}, \tag{1}$$

where $\tau = R_{CT}C_{DL}$. Provided that the electrochemical cell is used for the detection of biomolecular interactions in liquid solution, and according to Equation 1, the real part of the impedance monitors the bulk solution effect (i.e. the effect of the electrolyte alone) on the total impedance of the electrode; any biochemical interaction occurring on the sensing surface of the electrode, whether it is charge transfer ($R_{CT}$), or mass adsorption ($C_{DL}$) on the surface, affects the value of the imaginary part of the impedance. To continuously monitor changes in $Z_{imaginary}$ and/or $Z_{real}$ in real-time, a fixed-frequency AC signal, depending on the electrode design and its surface area, measurement setup and surface functionalisation, is applied by the potentiostat 3 to the working electrode. The selected frequency optimises the capacitive / resistive response of the cell, thereby maximising the sensitivity of the system. The response type depends on the biorecognition element used and/or the analyte(s) of interest. Thus, the response type is typically known by the operator of the electrochemical impedance system 1. The suggested range of frequency for sensing depending on the sensing signal is as follows:

- capacitive response only: 1 mHz to 1 kHz;
- capacitive and resistive response: 1 kHz to 100 kHz;
- resistive response only: > 100 kHz.

It's worth noting that the shape of the Bode diagram (and consequently the choice of the optimised working frequency) is highly dependent on the electrode design and the associated surface functionalisation. Hence, the choice of the operating frequency is significantly influenced by various factors, including the measurement setup, electrical connections, the internal low pass filter of the potentiostat, and the overall noise level in the system. While it is true that the impedance of the electrode remains independent of the amplitude of the input AC signal, it is worth noting that using a higher signal amplitude can be advantageous. This is particularly beneficial when the measured signal amplitude of the impedance approaches the limit of detection of the measurement setup, as it can lead to a reduction in the measurement noise level and an enhancement in the signal-to-noise ratio (SNR). Moreover, the amplitude of the input signal cannot be too high as the system should operate in small signal in order to extract impedance parameters. Therefore, the suggested range of the AC signal amplitude is below 100 mV peak-to-peak.

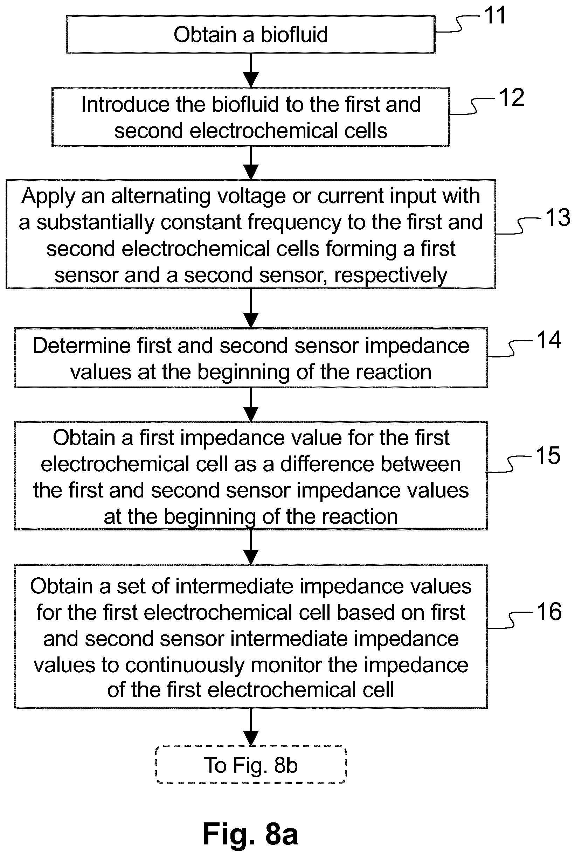

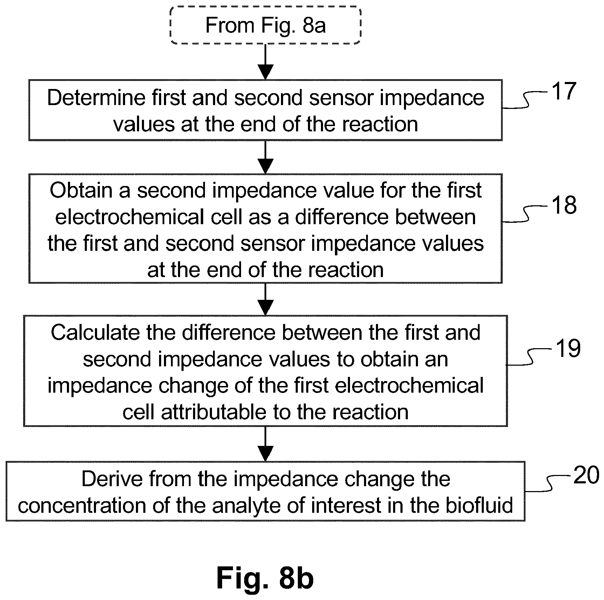

The flow chart of Figures 8a and 8b summarises the proposed method for monitoring biochemical interactions and/or sensing biomarkers in a biofluid by using an electrochemical impedance system 1 comprising in this example two electrochemical cells $2_a$, $2_r$, namely a first, active electrochemical cell $2_a$ and a second, reference electrochemical cell $2_r$. In this example, the first electrochemical cell forms a first sensor, which is an active sensor, while the second electrochemical cell forms a second sensor, which is a reference sensor. In this example, the first electrochemical cell is controlled by a first control and measurement arrangement 3, and the second electrochemical cell is controlled by a second control and measurement arrangement 3 for controlling operational conditions of the respective electrochemical cell and for measuring reactions in the respective electrochemical cell. However, it would be possible to use a single control and measurement arrangement for controlling both of the electrochemical cells.

In step 11, the biofluid is obtained or collected. The biofluid is a biological fluid obtained at least partially with a needle (in which case the biofluid may be interstitial fluid or blood), or by excretion (in which case the biofluid may be sweat, urine, or tears), or developed as a result of a pathological process (in which case the biofluid may be blister fluid). It is to be noted that sensors can be stored either in a dry state or immersed in a wet environment before the experiment. When opting for wet storage, it is important to replace the solution covering the sensor entirely with the specific biofluid of interest.

In step 12, the obtained or collected biofluid is applied or added to the first and second electrochemical cells $2_a$, $2_r$. In this example, ensuring the comprehensive coverage of all electrode surfaces within the electrochemical cell(s) by the biofluid is imperative. In step 13, the first and second control and measurement arrangements 3 apply an alternating voltage or current input with a substantially constant frequency to the first and second electrochemical cells, respectively. The applied input signal may be substantially identical for both the first and second electrochemical cells. The AC signal persists throughout the entire experiment, enabling real-time monitoring of biochemical interactions. In step 14, first and second sensor impedance values at the beginning of the reaction are determined by the control and measurement arrangements 3. More specifically, the first control and measurement arrangement determines a first sensor initial impedance value, and the second control and measurement arrangement determines a second sensor initial impedance value, which serves as an initial reference impedance value. The first and second sensor initial impedance values are obtained simultaneously or substantially simultaneously. In step 15, a first impedance value for the first electrochemical cell $2_a$ is determined as a difference between the first and second sensor impedance values at the beginning of the reaction. More specifically, the second sensor initial impedance value is subtracted from the first sensor initial impedance value. This determination may be carried out by one or both of the control and measurement arrangements or by another computing unit (under the assumption that the unit computing the difference has received the first and second sensor initial impedance values). The first impedance value of the first electrochemical cell is thus determined before, or as soon as a reaction between the biorecognition element(s) and one or more analytes of interest takes place.

In step 16, a set of first and second sensor intermediate impedance values are obtained, and a set of intermediate impedance values for the first electrochemical cell is obtained based on the first and second sensor intermediate impedance values to continuously measure the impedance of the first electrochemical cell for real-time monitoring of the biochemical reaction. In other words, a respective intermediate impedance value for the first electrochemical cell is obtained as a difference between a respective first sensor intermediate impedance value and a respective second sensor intermediate impedance value.

In step 17, first and second sensor impedance values at the end of the reaction are determined by the control and measurement arrangements 3. More specifically, the first control and measurement arrangement determines a first sensor end impedance value, and the second control and measurement arrangement determines a second sensor end impedance value, which serves as an end or final reference impedance value. The first and second sensor end impedance values are obtained simultaneously or substantially simultaneously. In step 18, a second impedance value for the first electrochemical cell is determined as a difference between the first and second sensor impedance values at the end of the reaction. More specifically, the second sensor end impedance value is subtracted from the first sensor end impedance value. This determination may be carried out by one or both of the control and measurement arrangements or by another computing unit (under the assumption that the unit computing the difference has received the first and second sensor end impedance values). The second impedance value of the first electrochemical cell is determined while maintaining a substantially constant input frequency after substantially all of the analyte or analytes of interest in the biofluid has/have reacted with the biorecognition element, or the biofluid has reached an equilibrium (in which case the number of vacant receptors of the biorecognition element remains substantially constant) or a saturation state (in which case there is more analyte than the receptors of the biorecognition element can capture). The time interval between the beginning of the reaction (or the moment of taking the first or initial impedance value) and the end of the reaction (or the moment of taking the second or end impedance value) in this case defines the length of the experiment, during which the frequency of the input signal is kept constant.

In step 19, the difference between the first and second impedance values to obtain an impedance change of the first electrochemical cell attributable to the reaction is calculated by one or both of the control and measurement arrangements or by another computing unit (under the assumption that the unit computing the impedance change has received the first and second impedance values). In step 20, the concentration of the analyte or analytes of interest in the biofluid is derived from the impedance change by one or both of the control and measurement arrangements or by another computing unit (under the assumption that the unit computing the difference has received the impedance change). This is possible assuming the volume or estimated volume of the biofluid in the first electrochemical cell is known. To obtain the concentration of analyte or analytes of interest in the biofluid, a calibration or mapping curve or table is used to map the value of impedance change to a specific concentration value. It is to be noted that in step 16 one or more first and second sensor intermediate impedance values may be measured to obtain intermediate impedance values for the first electrochemical cell to continuously monitor how the impedance value of the first electrochemical cell evolves. The above-described method also applies to single-sensor electrochemical impedance systems. If the electrochemical impedance system is a single-sensor system, then steps 14 and 17 are not needed, and in steps 15 and 18, the first and second impedance values are measured directly without calculating the difference between the first and second sensor impedance values.

To summarise the above teachings of the present invention, an electrochemical impedance system 1 and method are provided for monitoring biochemical interactions and/or sensing biomarkers in a biofluid. The system 1 comprises at least one electrochemical cell 2 with electrodes configured to come in contact with the biofluid, and a potentiostat 3 for controlling operational conditions of the electrochemical cell 2 and for measuring reactions in the electrochemical cell 2. The potentiostat 3 applies an alternating voltage or current input with a constant frequency during at least one experiment to the electrochemical cell 2, and measures an output signal of the electrochemical cell 2 to thereby measure at least an initial impedance value of the electrochemical cell 2 at the beginning of a reaction taking place in the electrochemical cell 2, and an end impedance value of the electrochemical cell 2 at the end of the reaction to measure an impedance change of the electrochemical cell 2 attributable to the reaction. The electrochemical impedance system 1 may form a wearable device, which in this case would be a modular miniaturised sensing platform that will offer unique health insight through the continuous and real-time analysis of one or more target analytes, such as proteins and hormones, at the surface of the skin (during critical health events for instance), to improve standard of care in a simple and minimally-invasive way.

While the invention has been illustrated and described in detail in the drawings and foregoing description, such illustration and description are to be considered illustrative or exemplary and not restrictive, the invention being not limited to the disclosed embodiments. Other embodiments and variants are understood and can be achieved by those skilled in the art when carrying out the claimed invention, based on a study of the drawings, the disclosure and the appended claims. Further variants may be obtained by combining the teachings of any of the designs explained above.

In the claims, the word "comprising" does not exclude other elements or steps, and the indefinite article "a" or "an" does not exclude a plurality. The mere fact that different features are recited in mutually different dependent claims does not indicate that a combination of these features cannot be advantageously used. Any reference signs in the claims should not be construed as limiting the scope of the invention.

**Claims**

1. A method for monitoring biochemical interactions and/or sensing biomarkers in a biofluid by using an electrochemical impedance system (1) comprising at least a first electrochemical cell (2, $2_a$) and a first control and measurement arrangement (3) for controlling operational conditions of the first electrochemical cell (2, $2_a$) and for measuring reactions in the first electrochemical cell (2), the control and measurement arrangement (3) being coupled to the first electrochemical cell (2, $2_a$) comprising a first working electrode (E1) and a first counter electrode (E2) both configured to come in contact with the biofluid comprising one or more analytes (5) of interest, at least one of the electrodes (E1, E2) being functionalised with a biorecognition element (4) sensitive to the one or more analytes of interest, the method comprising:

   - applying (13) an alternating voltage or current input with a substantially constant frequency to the first electrochemical cell (2, $2_a$);
   - determining (15) a first impedance value of the first electrochemical cell (2, $2_a$) before, or as soon as a reaction between the biorecognition element (4) and the one or more analytes of interest takes place;
   - determining (18) a second impedance value of the first electrochemical cell (2, $2_a$), while maintaining the substantially constant frequency, after substantially all of the one or more the analytes of interest in the biofluid has/have reacted with the biorecognition element (4), or the biofluid has reached an equilibrium or a saturation state;
   - calculating (19) from the first and second impedance values an impedance change of the first electrochemical cell (2, $2_a$) attributable to the reaction; and
   - deriving (20) from the impedance change the concentration of the one or more analytes (5) of interest in the biofluid.

2. The method according to claim 1, wherein the reaction is an electrochemical reaction, and/or the biorecognition element(s) (4) is/are specific to the one or more analytes (5) of interest, and/or wherein the one or more analytes of interest are molecular biomarkers, such as proteins, hormones, metabolites, nucleic acids, and/or whole cells.

3. The method according to claim 1 or 2, wherein the frequency is comprised between 1 mHz to 1 kHz for a capacitive response only, comprised between 1 kHz to 100 kHz for a capacitive and resistive response, or greater than 100 kHz for a resistive response only of the electrochemical impedance system (1).

4. The method according to any one of the preceding claims, wherein the biofluid is at least partially a biological fluid obtained at least partially with a needle, or by excretion, or developed as a result of a pathological process.

5. The method according to any one of the preceding claims, wherein the first and second impedance values are real impedance values, or imaginary impedance values, or a combination of both real and imaginary impedance values.

6. The method according to any one of the preceding claims, wherein the first working electrode (E1) and the first counter electrode (E2) and optionally a first reference electrode (E3) in the first electrochemical cell (2) collectively form a first sensor sensitive to the one or more analytes of interest, wherein the electrochemical impedance system (1) further comprises a second sensor insensitive to the one or more analytes of interest, and wherein the method further comprises:

   - determining (14) first and second sensor impedance values at the beginning of the reaction, wherein the first impedance value for the first electrochemical cell is obtained as a difference between the first and second sensor impedance values at the beginning of the reaction; and
   - determining (17) first and second sensor impedance values at the end of the reaction, wherein the second impedance value for the first electrochemical cell is obtained as a difference between the first and second sensor impedance values at the end of the reaction.

7. The method according to 6, wherein the first electrochemical cell (2) further comprises a second working electrode (E1r) and optionally the first reference electrode (E3), wherein the second sensor comprises the second working electrode (E1r) and the first counter electrode (E2) and optionally the first reference electrode (E3).

8. The method according to claim 6, wherein the electrochemical impedance system (1) further comprises a second electrochemical cell ($2_r$), which is separate from the first electrochemical cell ($2_a$), wherein the second electrochemical cell ($2_r$) comprises a second working electrode (E1r), a second counter electrode (E2r) and optionally a second reference electrode (E3r), wherein the second sensor comprises the second working electrode (E1r) and the second counter electrode (E2r) and optionally the second reference electrode (E3r).

9. An electrochemical impedance system (1) for monitoring biochemical interactions and/or sensing biomarkers in a biofluid, the electrochemical impedance system (1) comprising:

- a first electrochemical cell (2, $2_a$) comprising a first working electrode (E1) and a first counter electrode (E2), both the first working electrode (E1) and the first counter electrode (E2) being configured to come in contact with the biofluid, at least one of the first working electrode (E1) and the first counter electrode (E2) being functionalised with a biorecognition element (4) for capturing one or more analytes (5) of interest in the biofluid; and
- a first control and measurement arrangement (3) for controlling operational conditions of the first electrochemical cell (2, $2_a$) and for measuring reactions in the first electrochemical cell (2, $2_a$), the control and measurement arrangement (3) being coupled to the first electrochemical cell (2, $2_a$) and configured to apply an alternating voltage or current input with a substantially constant frequency during at least one experiment to one of the first working electrode (E1) and the first counter electrode (E2), the control and measurement arrangement (3) being further configured to measure an output signal at the other one of the first working electrode (E1) and the first counter electrode (E2) to thereby measure at least an initial impedance value of the first electrochemical cell (2, $2_a$) at the beginning of a reaction between the biorecognition element (4) and the one or more analytes (5) of interest and an end impedance value of the first electrochemical cell (2, $2_a$) at the end of the reaction to measure an impedance change of the first electrochemical cell (2, $2_a$) attributable to the reaction taking place in the first electrochemical cell (2, $2_a$).

10. The electrochemical impedance system (1) according to claim 9, wherein the electrochemical impedance system (1) is a single electrochemical cell system, and wherein the first electrochemical cell (2, $2_a$) optionally comprises a first reference electrode (E3).

11. The electrochemical impedance system (1) according to claim 9 or 10, wherein the first electrochemical cell (2, $2_a$) further comprises a second working electrode (E1r), wherein the first working electrode (E1) and the first counter electrode (E2) and optionally the first reference electrode (E3) collectively form a first sensor sensitive to the one or more analytes (5) of interest, and the second working electrode (E1r) and the first counter electrode (E2) and optionally the first reference electrode (E3) collectively form a second sensor insensitive to the analytes of interest, and wherein a respective impedance value of the first electrochemical cell (2, $2_a$) is obtained as a difference between a respective first sensor impedance value and a respective second sensor impedance value.

12. The electrochemical impedance system (1) according to claim 9, wherein the electrochemical impedance system (1) further comprises a second electrochemical cell ($2_r$) comprising a second working electrode (E1r) and a second counter electrode (E2r), wherein at least the first working electrode (E1) and the first counter electrode (E2) collectively form a first sensor sensitive to the one or more analytes (5) of interest, and at least the second working electrode (E1r) and the second counter electrode (E2r) collectively form a second sensor insensitive to the one or more analytes (5) of interest, and wherein a respective impedance value of the first electrochemical cell (2, $2_a$) is obtained as a difference between a respective first sensor impedance value and a respective second sensor impedance value.

13. The electrochemical impedance system (1) according to claim 12, wherein the first electrochemical cell ($2_a$) further comprises a first reference electrode (E3) and the second electrochemical cell ($2_r$) further comprises a second reference electrode (E3r), and wherein the first working electrode (E1), the first counter electrode (E2), and the first reference electrode (E3) collectively form the first sensor sensitive to the one or more analytes (5) of interest, and the second working electrode (E1r), the second counter electrode (E2r), and the second reference electrode (E3r) collectively form the second sensor insensitive to the one or more analytes (5) of interest.

14. The electrochemical impedance system (1) according to any one of claims 9 to 13, wherein the biorecognition element (4) comprises, or is composed of biological material, or the biorecognition element (4) comprises, or is composed of biologically derived material, or the biorecognition element (4) comprises, or is composed of bio-mimic material, or the biorecognition element (4) comprises, or is composed of any combination of biological material, biologically derived material, and/or bio-mimic material.

15. The electrochemical impedance system (1) according to claim 14, wherein the biological material comprises one or more antibodies, enzymes, nucleic acids, and/or cell receptors, the biologically derived material comprises one or more aptamers, and/or recombinant antibodies, or the bio-mimic material comprises one or more molecularly imprinted polymers and/or synthetic catalysts.

**Fig. 1**

EP 4 600 639 A1

**Fig. 2**

**Fig. 3**

**Fig. 4**

**Fig. 5**

**Fig. 6**

Fig. 7

EP 4 600 639 A1

| Obtain a biofluid | 11 |

| Introduce the biofluid to the first and second electrochemical cells | 12 |

| Apply an alternating voltage or current input with a substantially constant frequency to the first and second electrochemical cells forming a first sensor and a second sensor, respectively | 13 |

| Determine first and second sensor impedance values at the beginning of the reaction | 14 |

| Obtain a first impedance value for the first electrochemical cell as a difference between the first and second sensor impedance values at the beginning of the reaction | 15 |

| Obtain a set of intermediate impedance values for the first electrochemical cell based on first and second sensor intermediate impedance values to continuously monitor the impedance of the first electrochemical cell | 16 |

To Fig. 8b

**Fig. 8a**

From Fig. 8a

Determine first and second sensor impedance values at the end of the reaction ⟿17

Obtain a second impedance value for the first electrochemical cell as a difference between the first and second sensor impedance values at the end of the reaction ⟿18

Calculate the difference between the first and second impedance values to obtain an impedance change of the first electrochemical cell attributable to the reaction ⟿19

Derive from the impedance change the concentration of the analyte of interest in the biofluid ⟿20

# Fig. 8b

Europäisches Patentamt

European Patent Office

Office européen des brevets

# EUROPEAN SEARCH REPORT

**Application Number**

EP 24 15 6341

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | RAJESH ET AL: "Single Frequency Impedance Analysis on Reduced Graphene Oxide Screen-Printed Electrode for Biomolecular Detection", APPLIED BIOCHEMISTRY AND BIOTECHNOLOGY, HUMANA PRESS INC, NEW YORK, vol. 183, no. 2, 22 May 2017 (2017-05-22), pages 672-683, XP036334058, ISSN: 0273-2289, DOI: 10.1007/S12010-017-2510-8 [retrieved on 2017-05-22] | 1-5,9, 10,14,15 | INV. G01N27/30 G01N33/543 G01N33/487 A61B5/14 |
| Y | * abstract * * page 673 * * figure 2 * * page 678 - page 680 * | 6-8, 11-13 | |
| X | Amir Mohsen Aliakbar: "HANDHELD IMPEDANCE BASED BIOSENSOR SYSTEM FOR GLUCOSE MONITORING", , 1 August 2009 (2009-08-01), XP055327952, Retrieved from the Internet: URL:https://www.google.nl/url?sa=t&rct=j&q =&esrc=s&source=web&cd=8&ved=0ahUKEwiToNSi me7QAhVXNFAKHRRaAo4QFghUMAc&url=http://dig itool.library.mcgill.ca/thesisfile66772.pd f&usg=AFQjCNECvwlvtuWzt9311NsMS49IqBg5ZQ [retrieved on 2024-06-19] * Chapter 2.1.3, chapter 5.1 * | 1,9 | |

| | |
|---|---|
| TECHNICAL FIELDS SEARCHED (IPC) | |
| G01N A61B | |

-/--

The present search report has been drawn up for all claims

| Place of search | Date of completion of the search | Examiner |
|---|---|---|
| Munich | 20 June 2024 | Klein, Marc-Oliver |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 24 15 6341

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ÇIGDEM SAYIKLI SIMSEK ET AL: "An ITO Based Disposable Biosensor for Ultrasensitive Analysis of Retinol Binding Protein", ELECTROANALYSIS, VHC PUBLISHERS, INC, US, vol. 26, no. 2, 23 January 2014 (2014-01-23), pages 328-339, XP071937497, ISSN: 1040-0397, DOI: 10.1002/ELAN.201300443 * page 337 * * figure 6 * | 1,9 | |
| Y | ROSATI GIULIO ET AL: "Silver nanoparticles inkjet-printed flexible biosensor for rapid label-free antibiotic detection in milk", SENSORS AND ACTUATORS B: CHEMICAL, ELSEVIER BV, NL, vol. 280, 10 October 2018 (2018-10-10), pages 280-289, XP085537629, ISSN: 0925-4005, DOI: 10.1016/J.SNB.2018.09.084 * p. 286, right column, last two lines - p. 287 left column * | 6-8, 11-13 | |

TECHNICAL FIELDS
SEARCHED (IPC)

The present search report has been drawn up for all claims

| Place of search | Date of completion of the search | Examiner |
|---|---|---|
| Munich | 20 June 2024 | Klein, Marc-Oliver |

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LASSERRE et al.** SARS-CoV-2 aptasensors based on electrochemical impedance spectroscopy and low-cost gold electrode substrates. *Anal Chem.*, 2022, vol. 94, 2126-2133
- **VASANTHAM et al.** Paper based point of care immunosensor for the impedimetric detection of cardiac troponin I biomarker. *Biomed Microdevices*, 2019, vol. 22, 6

- **AYDIN et al.** An impedimetric immunosensor for highly sensitive detection of IL-8 in human serum and saliva samples: a new surface modification method by 6-phosphonohexanoic acid for biosensing applications. *Anal Biochem*, 2018, vol. 554, 44-52
- Selective and ultrasensitive electrochemical immunosensing of NSE cancer biomarker in human serum using epoxy-substituted poly(pyrrole) polymer modified disposable ITO electrode. *Sens Actuators B Chem.*, 2020, vol. 306, 127613